# EUROPEAN PATENT APPLICATION

(11) **EP 4 509 503 A1**
(43) Date of publication of application: **19.02.2025**
(21) Application number: 23787710.5
(22) Date of filing: 11.04.2023
(51) Int. Cl.: C07D 403/12, C07D 207/26, A61K 31/405, A61K 31/404, A61P 31/14

(54) **COMPOUND AND APPLICATION THEREOF IN PREPARATION OF MEDICINE FOR TREATING ENTEROVIRUS-RELATED DISEASES**

(30) Priority: 11.04.2022 WO PCT/CN2022/086047
(71) Applicant: ShanghaiTech University, Shanghai 201210 (CN)
(72) Inventor: YANG, Haitao, Shanghai 201210 (CN); ZHOU, Hao, Shanghai 201210 (CN); DUAN, Yinkai, Shanghai 201210 (CN); WANG, Haofeng, Shanghai 201210 (CN); YANG, Xiuna, Shanghai 201210 (CN); RAO, Zihe, Shanghai 201210 (CN)
(74) Representative: Greaves Brewster LLP
(86) International application number: PCT/CN2023/087646
(87) International publication number: WO 2023/198067

(57) **Abstract**

Disclosed are a compound and an application thereof in the preparation of a medicine for treating enterovirus-related diseases. The compound is the compound represented below by formula I, and can significantly inhibit the 3C protease activity of various enteroviruses. At present, no specific medicine aimed at human enteroviruses has been approved to come to market, and the use of the solution of the present invention can compensate for the insufficiencies of the prior art.

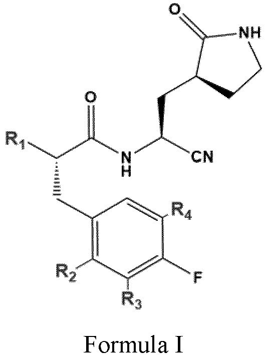

## Description

### TECHNICAL FIELD

The present invention belongs to the field of virus therapy and relates to a compound and an application thereof in the preparation of a medicine for treating enterovirus-related diseases.

### BACKGROUND

Enterovirus is a ubiquitous virus with a wide variety of strains and diverse infection symptoms. The enteroviruses belong to the Picornaviridae family, and include poliovirus, coxsackievirus, echovirus, enterovirus, and rhinovirus¹. The enterovirus is transmitted mainly via fecal-oral route or respiratory droplets. Enterovirus infections often manifest as mild respiratory illnesses (common colds), and thus is often overlooked. But as the most common infectious disease in humans, they can also lead to severe conditions such as poliomyelitis, aseptic meningitis, conjunctivitis, pericarditis, hand-foot-and-mouth disease, and even paralysis^{2, 3}. With the exception of poliovirus vaccines, there are currently no marketed vaccines or antiviral medications available for most enterovirus infections.

The genome of enterovirus is a single strand of positive-sense RNA, about 7.5 kb in length, which mainly encodes structural proteins required for viral packaging and non-structural proteins crucial for viral replication and transcription. Due to the low-fidelity nature of viral replication and frequent recombination, the genome of enterovirus is highly mutable⁴. The viral polyprotein, encoded by an open reading frame, is divided into three regions, P1-P3. The P1 region contains four structural proteins (VP1-VP4), while the P2 and P3 regions contain seven non-structural proteins (2A-2C and 3A-3D). The virus can complete normal transcription and replication only when these functional subunits are cleaved into independent protein units by virus-coded proteases. Upon host cell infection, the P1 region, which encodes capsid structural protein, is translated first. Subsequently, during the translation of the P2 and P3 regions, 2A and 3C proteases are encoded and released from the polyprotein. These proteases are responsible for further polyprotein cleavage, with 2A protease having two cleavage sites and 3C protease having eight⁵. It can be seen that the 3C protease plays a pivotal regulatory role in the transcription and replication of the virus, and thus has become the focus of research⁶. The 3C protease of the enterovirus is a multifunctional cysteine protease, which belongs to proteases of a chymotrypsin-related endopeptidase family⁷. In different species of enteroviruses, the 3C proteases share the sequence similarity of about 50-75%⁶. Residues of the catalytic triplet are completely conserved throughout the enteroviruses. In addition, amino acid residues of a protein surrounding catalytic residues are more conserved than the rest of the protein, suggesting similar mechanisms involving the sequence specificity and cleavage between enterovirus proteases.

The seasonality and prevalence of rhinovirus (HRV) make it the most well-known enterovirus, causing a variety of complications in children, the elderly, and immunocompromised people⁸. HRV infection can cause up to 80% of viral asthma exacerbations, with up to 16 deaths per week in Australia and 25 deaths per week in the United States⁹. Although poliovirus has been eliminated by vaccines, the development for HRV vaccines is not feasible due to the excessive number (more than 100) of HRV serotypes. AG7088 is a 3C protease inhibitor with significant antiviral activity against a variety of human rhinovirus serotypes and negligible inhibitory activity against mammalian cysteine and serine proteases, including cathepsin B, elastase, chymotrypsin, trypsin, thrombin, and calpain¹⁰. However, due to the low bioavailability of AG7088, scientists have been trying to develop a successful specific medicine based on the AG7088 mechanism of action for many years, but no such drug has been developed yet¹¹.

### BRIEF SUMMARY OF THE INVENTION

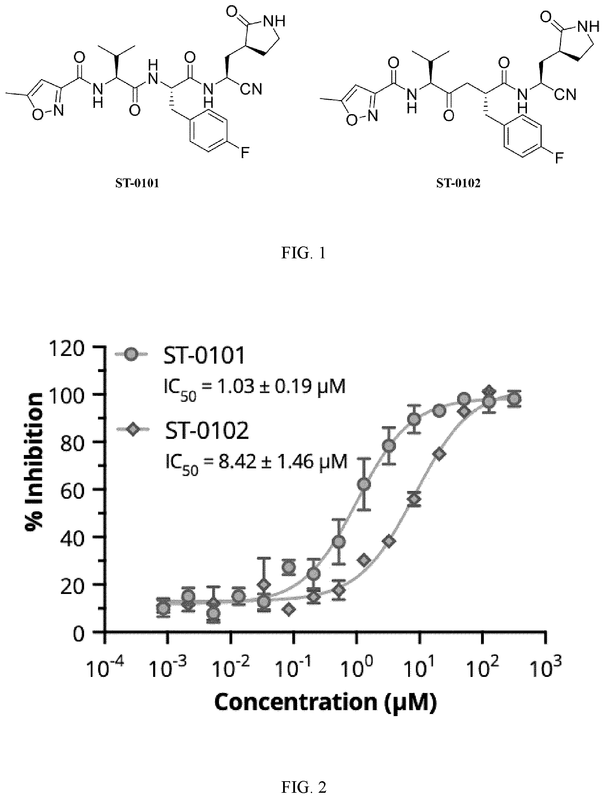

The present invention addresses a critical gap in existing therapeutic options for diseases caused by members of the Enterovirus genus. Specifically, the present invention provides a compound, and an application thereof in the development of treatments for enterovirus-related diseases. An object of the present invention is to provide a treatment solution for diseases caused by enterovirus infections. ST-0101 and ST-0102 in the present invention can be used for the treatment of diseases caused by rhinovirus, as well as major infectious diseases caused by other enteroviruses, such as poliovirus, coxsackievirus, echovirus, and enterovirus. ST-0101 and ST-0102 (shown in FIG. 1) may reversibly bind to a cysteine residue (C147) of a HRV 3C protease. It is found in the present invention that ST-0101 and ST-0102 may be used for the treatment of related diseases caused by enteroviruses through in vitro enzyme activity experiments and crystal structure analysis of protein complexes.

In order to solve the above technical problem, a first aspect of the present invention provides a compound, wherein the compound is represented below by formula I:

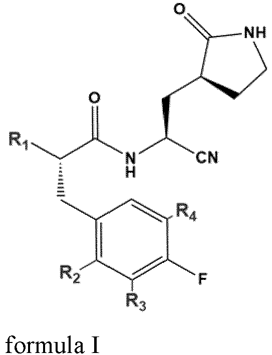

wherein, R1 is selected from: 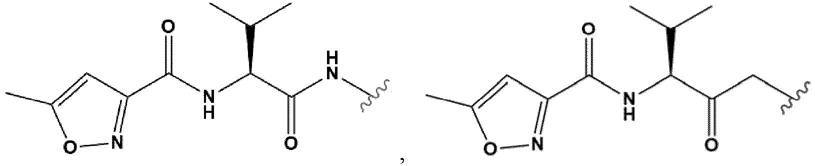 or 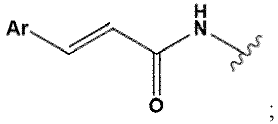 R2, R3 and R4 are selected from H and halogen.

In some preferred embodiments, in the compound, when R1 is 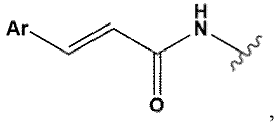 Ar is 3,4-(OCH₂)C₆H₃, 4-Me₂NC₆H₄, C₆H₅, 4-MeC₆H₄, 4-Cl,2-FC₆H₃ or 5-Methyl-3-isoxazole; and R2, R3 and R4 are H, F or CF3.

In some more preferred embodiment, the compound is represented by formula II or III:

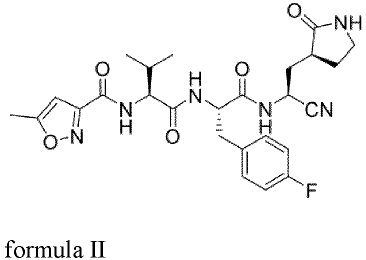

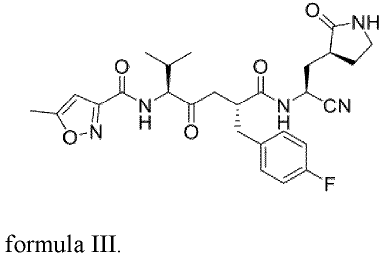

In order to solve the above technical problems, a second aspect of the present invention provides a pharmaceutical composition, the pharmaceutical composition comprising: the compound in the first aspect of the present invention or a pharmaceutically acceptable salt thereof; and preferably, further comprising a pharmaceutically acceptable diluent or carrier.

In order to solve the above technical problems, a third aspect of the present invention provides a use of the compound in the first aspect of the present invention or the pharmaceutical composition in the second aspect of the present invention in preparation of an inhibitor for an enterovirus protease.

In some preferred embodiments, the enterovirus protease is selected from a 2A protease, a 2B protease, a 2C protease, a 3A protease, a 3B protease, a 3C protease and a 3D protease; and/or, the enterovirus protease is a protease of rhinovirus, poliovirus, coxsackievirus, echovirus or enterovirus.

In some more preferred embodiments, the enterovirus protease is a 3C protease of rhinovirus, poliovirus, coxsackievirus, echovirus or enterovirus.

Preferably, the 3C protease is a 3C protease of rhinovirus HRV-A2 or HRV-B14, poliovirus Poliovirus type 1, enterovirus EV71, echovirus Echovirus1 or coxsackievirus CVB3.

In order to solve the above technical problems, a fourth aspect of the present invention provides a use of the compound in the first aspect of the present invention or the pharmaceutical composition in the second aspect of the present invention in preparation of a medicine for treating intestinal-related diseases.

In some preferred embodiments, the intestinal-related diseases are diseases caused by enteroviruses.

Preferably, the enteroviruses comprise rhinovirus, poliovirus, coxsackievirus, echovirus, and enterovirus.

In some more preferred embodiments, the rhinovirus comprises HRV-A2 and HRV-B14, the coxsackievirus comprises CVB3, the echovirus comprises Echovirus1, the enterovirus comprises EV71, and the poliovirus comprises Poliovirus type 1.

In order to solve the above technical problems, a fifth aspect of the present invention provides a method for treating or preventing intestinal-related diseases, which comprises administrating a subject with an effective amount of the compound in the first aspect of the present invention or the pharmaceutical composition in the second aspect of the present invention.

In some preferred embodiments, the intestinal-related diseases are diseases caused by enteroviruses.

Preferably, the enteroviruses comprise rhinovirus, poliovirus, coxsackievirus, echovirus, and enterovirus.

More referably, the rhinovirus comprises HRV-A2 and HRV-B14, the coxsackievirus comprises CVB3, the echovirus comprises Echovirus1, the enterovirus comprises EV71, and the poliovirus comprises Poliovirus type 1.

In some preferred embodiments, the method further comprises: administrating other medicines for the treatment or prevention of intestinal-related diseases.

Preferably, the other medicines are inhibitors that inhibit 3C proteases.

More preferably, the inhibitors that inhibit the 3C proteases comprise AG7088 or other AG7088 derivatives other than the compound in the first aspect of the present invention.

In order to solve the above technical problems, a sixth aspect of the present invention provides a method for *in vitro* inhibition or detection of a 3C protease for non-diagnostic purposes, or a method for *in vivo* inhibition or detection of a 3C protease; and the method comprises: contacting the 3C protease with an effective amount of the compound in the first aspect of the present invention or the pharmaceutical composition in the second aspect of the present invention. An application scenario of the method for *in vitro* inhibition or detection of the 3C protease for non-diagnostic purposes is, for example, in scientific research, in which the compound of the present invention comes into contact with a sample in a laboratory to determine whether the sample contains the 3C protease.

In some preferred embodiments, the 3C protease is a 3C protease of rhinovirus, poliovirus, coxsackievirus, echovirus or enterovirus.

The present invention has the positive progressive effect in that:

The compound disclosed by the present invention, e.g., the compound represented by formula II or III, can significantly inhibit the 3C protease activities of various enteroviruses. At present, no specific medicine aimed at human enteroviruses has been approved to come to market, and the treatment protocol for treating intestinal-related diseases with the compound represented by formula II or III can compensate for the insufficiencies of the prior art.

### BRIEF DESCRIPTION OF THE DRAWINGS

FIG. 1 shows molecular formulas of ST-0101 and ST-0102.
FIG. 2 shows that both ST-0101 and ST-0102 have significant inhibitory activities against a 3C protease of rhinovirus HRV-A2.
FIG. 3 shows that ST-0101 has a significant inhibitory activity against a 3C protease of rhinovirus HRV-B14.
FIG. 4 shows that both ST-0101 and ST-0102 have significant inhibitory activities against a 3C protease of Poliovirus type 1.
FIG. 5 shows that both ST-0101 and ST-0102 have significant inhibitory activities against a 3C protease of enterovirus EV71.
FIG. 6 shows that both ST-0101 and ST-0102 have significant inhibitory activities against a 3C protease of Echovirus1.
FIG. 7 shows that both ST-0101 and ST-0102 have significant inhibitory activities against a 3C protease of coxsackievirus CVB3.
FIG. 8 shows a reversible covalent interaction of a 3C protease of HRV and an inhibitor ST0102.

### DETAILED DESCRIPTION OF THE PREFERRED EMBODIMENT

The present invention is further described below by means of examples, but is not limited in the above example scope. The experimental methods in the following examples in which specific conditions were not specified were selected according to conventional methods and conditions, or according to the product manual.

### Example 1

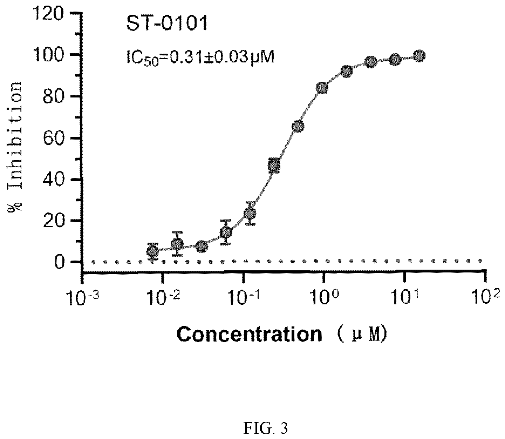

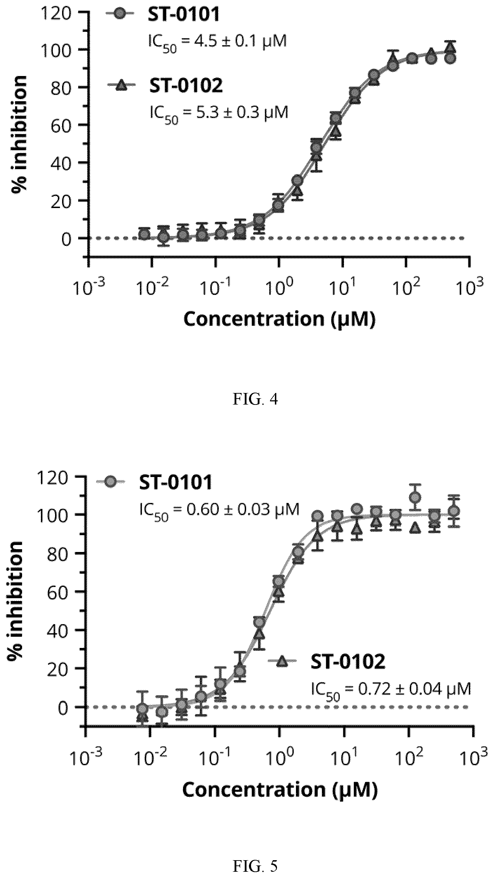

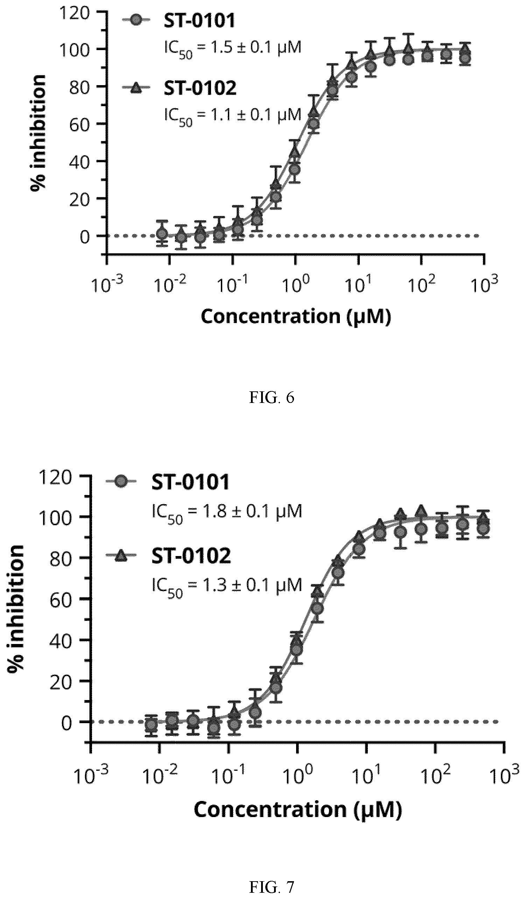

Through *in vitro* enzyme activity experiments, it was found that ST-0101 and ST-0102 can significantly inhibit the activities of 3C proteases of rhinovirus (HRV-A2, HRV-B14), poliovirus (Poliovirus type 1), enterovirus (EV71), echovirus (Echovirus1), and coxsackievirus (CVB3) (FIGS. 2 to 7).

A fluorescent substrate used to measure inhibitory effects of ST-0101 and ST-0102 on the 3C proteases of the above-mentioned viruses was Dabcyl-Leu-Glu-Val-Leu-Phe-Gln-Gly-Pro-Lys (5-FAM)-NH₂ (SEQ ID NO: 3). This amino acid sequence of the substrate was mainly designed based on classic recognition sequences of the 3C proteases. Both ends of a short peptide were connected to a fluorescent group 5-FAM and a quenching group Dabcyl respectively. After the short peptide was cleaved by the 3C protease, the quenching group and the fluorescent group were separated, and a fluorescent signal from the fluorescent group can be detected. Therefore, when the 3C protease reacted with the substrate, a rate at which the fluorescence intensity increased can reflect an enzymatic reaction rate of the 3C protease. When different concentrations of inhibitory molecules were added, the enzyme activity was inhibited to different degrees and the increase rate of the fluorescence intensity was also inhibited. IC50 curves of inhibitors can be obtained by measuring the corresponding enzyme activities of inhibitors of different concentrations, with the fluorescence increasing rate being 100% enzymatic activity without the addition of inhibitor molecules, and a fluorescence increasing rate being 0% enzymatic activity without the addition of enzymes (substrate molecules may degrade themselves at a very slow rate). A buffer solution system used in this experiment includes 20 mM Tris pH 8.0, 150 mM NaCl, 0.1 mg/ml bovine serum albumin (BSA), and 0.01% (v/v) TritonX-100, with the substrate used having a concentration of 20 µM and a protein used having a concentration of 0.5 µM.
HRV-A2 3C amino acid sequence (SEQ ID NO: 1): 
GPEEEFGMSLIKHNSCVITTENGKFTGLGVYDRFVVVPTHADPGKEIQVDGI
TTKVIDSYDLYNKNGIKLEITVLKLDRNEKFRDIRRYIPNNEDDYPNCNLALLANQPE
PTIINVGDVVSYGNILLSGNQTARMLKYSYPTKSGYCGGVLYKIGQVLGIHVGGNGR
DGFSAMLLRSYFTDVQ
HRV-B14 3C amino acid sequence (SEQ ID NO: 2): 
GPNTEFALSLLRKNIMTITTSKGEFTGLGIHDRVCVIPTHAQPGDDVLVNGQK
IRVKDKYKLVDPENINLELTVLTLDRNEKFRDIRGFISEDLEGVDATLVVHSNNFTNTIL
EVGPVTMAGLINLSSTPTNRMIRYDYATKTGQCGGVLCATGKIFGIHVGGNGRQGFS
AQLKKQYFVEKQ
Poliovirus type 1 3C amino acid sequence (SEQ ID NO: 4): 
GPGFDYAVAMAKRNIVTATTSKGEFTMLGVHDNVAILPTHASPGESIVIDGKE
VEILDAKALEDQAGTNLEITIITLKRNEKFRDIRPHIPTQITETNDGVLIVNTSKYPNMY
VPVGAVTEQGYLNLGGRQTARTLMYNFPTRAGQCGGVITCTGKVIGMHVGGNGSH
GFAAALKRSYFTQSQ
EV71 3C amino acid sequence (SEQ ID NO: 5): 
GPSLDFALSLLRRNIRQVQTDQGHFTMLGVRDRLAVLPRHSQPGKTIWIEHK
LVNVLDAVELVDEQGVNLELTLITLDTNEKFRDITKFIPENISTASDATLVINTEHMPSM
FVPVGDVVQYGFLNLSGKPTHRTMMYNFPTKAGQCGGVVTSVGKVIGIHIGGNGRQ
GFCAGLKRSYFASEQ
Echovirus 1 3C amino acid sequence (SEQ ID NO: 6): 
GPAFEFAVAMMKRNASTVKTEYGEFTMLGIYDRWAVLPRHAKPGPTILMND
QEVGVLDAKELVDKDGTNLELTLLKLNRNEKFRDIRGFLAREEAEVNEAVLAINTSK
 
FPNMYIPVGQVTDYGFLNLGGTPTKRMLMYNFPTRAGQCGGVLMSTGKVLGIHVG
GNGHQGFSAALLRHYFNEEQ
CVB3 3C amino acid sequence (SEQ ID NO: 7):

GPAFEFAVAMMKRNSSTVKTEYGEFTMLGIYDRWAVLPRHAKPGPTILMND
QEVGVLDAKELVDKDGTNLELTLLKLNRNEKFRDIRGFLAKEEVEVNEAVLAINTSK
FPNMYIPVGQVTEYGFLNLGGTPTKRMLMYNFPTRAGQCGGVLMSTGKVLGIHVG
GNGHQGFSAALLKHYFNDEQ

### Example 2

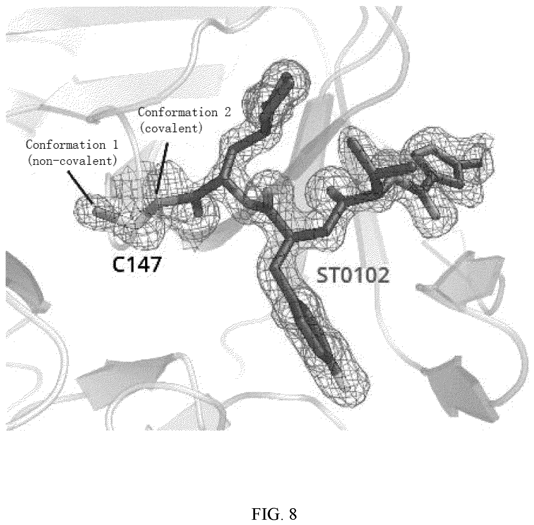

In the present invention, 1.54 angstrom high-resolution structures of a HRV 3C protease and an inhibitor ST-0102 were analyzed. As shown in FIG. 8, it can be seen that a reversible covalent bond was formed between C147 in the active center of the protease and the inhibitor. Crystals of the complex of the HRV 3C protease and the inhibitor ST-0102 were screened by a Hampton Research Index kit using a sitting drop method. X-ray diffraction data of the crystals was collected at the Shanghai Synchrotron Radiation Facility. A high-resolution structure of the complex of the HRV 3C protease and the inhibitor ST-0102 was finally obtained by processing the diffraction data of the crystals using an XDS software package, taking 5FX6 in the RCSB database as a corresponding initial model, performing molecular substitution using a Phenix.phaser program, and performing repeated corrections using Phenix.refine and Coot software.

Since the substrate-binding pocket of the 3C protease of the enterovirus was highly conserved, inhibitors (ST-0101 & ST-0102) targeting its binding pocket had broad-spectrum anti-enterovirus effects. ST-0101 & ST-0102 in the present invention may be used for the treatment of major infectious diseases caused by rhinovirus, poliovirus, coxsackievirus, echovirus, enterovirus, etc.

Although the specific embodiments of the present invention are described above, those skilled in the art should understand that these are only illustrations, and that various changes or modifications may be made to these embodiments without deviating from the principle and substance of the present invention. Therefore, the protection scope of the present invention should be defined by the appended claims.

### References:

1. O. S. Nikonov, E. S. Chernykh, M. B. Garber, E. Y. Nikonova, Enteroviruses: Classification, Diseases They Cause, and Approaches to Development of Antiviral Drugs. Biochemistry (Mosc) 82, 1615-1631 (2017).
2. L. Royston, C. Tapparel, Rhinoviruses and Respiratory Enteroviruses: Not as Simple as ABC. Viruses 8, (2016).
3. B. Jubelt, H. L. Lipton, Enterovirus/picornavirus infections. Handb Clin Neurol 123, 379-416 (2014).
4. M. Nikolaidis et al., Large-scale genomic analysis reveals recurrent patterns of intertypic recombination in human enteroviruses. Virology 526, 72-80 (2019).
5. O. H. Laitinen et al., Enteroviral proteases: structure, host interactions and pathogenicity. Rev Med Virol 26, 251-267 (2016).
6. M. Laajala, D. Reshamwala, V. Marjomaki, Therapeutic targets for enterovirus infections. Expert Opin Ther Tar 24, 745-757 (2020).
7. J. Seipelt et al., The structures of picornaviral proteinases. Virus Res 62, 159-168 (1999).
8. C. Esneau, A. C. Duff, N. W. Bartlett, Understanding Rhinovirus Circulation and Impact on Illness. Viruses 14, (2022).
9. L. M. Jensen, E. J. Walker, D. A. Jans, R. Ghildyal, Proteases of human rhinovirus: role in infection. Methods Mol Biol 1221, 129-141 (2015).
10. D. A. Matthews et al., Structure-assisted design of mechanism-based irreversible inhibitors of human rhinovirus 3C protease with potent antiviral activity against multiple rhinovirus serotypes. Proc Natl Acad Sci U S A 96, 11000-11007 (1999).
11. K. Namoto et al., Structure-based design and synthesis of macrocyclic human rhinovirus 3C protease inhibitors. Bioorg Med Chem Lett 28, 906-909 (2018).

## Claims

1. A compound, wherein the compound is represented below by formula I: 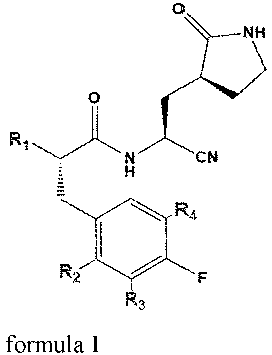 wherein, R1 is selected from: 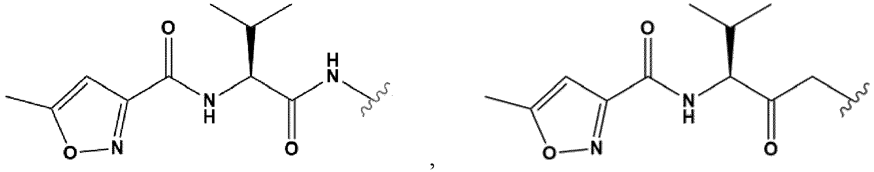 or 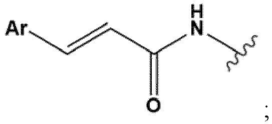 R2, R3 and R4 are selected from H and halogen.

2. The compound according to claim 1, wherein in the compound, when R1 is 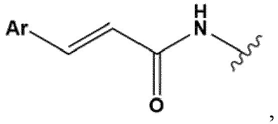 Ar is 3,4-(OCH₂)C₆H₃, 4-Me₂NC₆H₄, C₆H₅, 4-MeC₆H₄, 4-Cl,2-FC₆H₃ or 5-Methyl-3-isoxazole; and R2, R3 and R4 are H, F or CF3.

3. The compound according to claim 1, wherein the compound is represented by formula II or III:

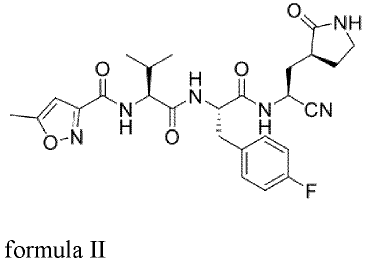

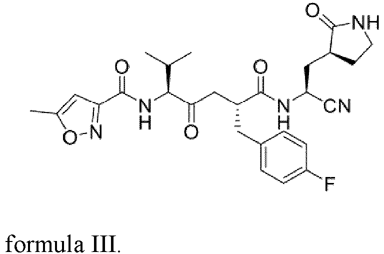

4. A pharmaceutical composition, comprising the compound according to any one of claims 1 to 3 or a pharmaceutically acceptable salt thereof.

5. The pharmaceutical composition according to claim 4, further comprising a pharmaceutically acceptable diluent or carrier.

6. A use of the compound according to any one of claims 1 to 3 or the pharmaceutical composition according to claim 4 or 5 in preparation of an inhibitor for an enterovirus protease.

7. The use according to claim 6, wherein the enterovirus protease is selected from a 2A protease, a 2B protease, a 2C protease, a 3A protease, a 3B protease, a 3C protease and a 3D protease; and/or, the enterovirus protease is a protease of rhinovirus, poliovirus, coxsackievirus, echovirus or enterovirus.

8. The use according to claim 7, wherein the enterovirus protease is a 3C protease of rhinovirus, poliovirus, coxsackievirus, echovirus or enterovirus.

9. The use according to claim 8, wherein the 3C protease is a 3C protease of rhinovirus HRV-A2 or HRV-B14, poliovirus Poliovirus type 1, enterovirus EV71, echovirus Echovirus1 or coxsackievirus CVB3.

10. A use of the compound according to any one of claims 1 to 3 or the pharmaceutical composition according to claim 4 or 5 in preparation of a medicine for treating intestinal-related diseases.

11. The use according to claim 10, wherein the intestinal-related diseases are diseases caused by enteroviruses.

12. The use according to claim 11, wherein the enteroviruses comprise rhinovirus, poliovirus, coxsackievirus, echovirus or enterovirus.

13. The use according to claim 12, wherein the rhinovirus comprises HRV-A2 and HRV-B14, the coxsackievirus comprises CVB3, the echovirus comprises Echovirus1, the enterovirus comprises EV71, and the poliovirus comprises Poliovirus type 1.

14. A method for *in vitro* inhibition or detection of a 3C protease for non-diagnostic purposes, comprising: contacting the 3C protease with an effective amount of the compound according to any one of claims 1 to 3 or the pharmaceutical composition according to claim 4 or 5 *in vitro.*

15. The method according to claim 14, wherein the 3C protease is a 3C protease of rhinovirus, poliovirus, coxsackievirus, echovirus or enterovirus.
